# FASCICULE DE BREVET EUROPEEN

(11) **EP 1 012 227 B1**
(45) Date de publication et mention de la délivrance du brevet: **16.10.2002**
(21) Numéro de dépôt: 99929480.4
(22) Date de dépôt: 13.07.1999
(51) Int. Cl.: C12M 1/00, A61J 1/00

(54) **POCHES SOUPLES POUR LE TRANSPORT DE PRODUITS FLUIDES BIO-PHARMACEUTIQUES**
Flexible Beutel für flüssige biopharmazeutische Produkte
FLEXIBLE BAGS FOR TRANSPORTING BIOPHARMACEUTICAL FLUID PRODUCTS

(30) Priorité: 16.07.1998 FR 9809244
(43) Date de publication de la demande: 28.06.2000
(73) Titulaire: STEDIM S.A., 13781 Aubagne Cedex (FR)
(72) Inventeur: VALLOT, Bernard, F-13012 Marseille (FR)
(74) Mandataire: Perin, Georges
(86) Numéro de dépôt international: FR9901711
(87) Numéro de publication internationale: WO00004131

(56) Documents cités:
- EP-A- 0 069 807
- EP-A- 0 282 230
- EP-A- 0 567 210
- WO-A-98/13469
- FR-A- 2 552 330
- US-A- 4 119 267
- US-A- 4 801 486
- US-A- 4 837 047
- US-A- 4 968 624

## Description

La présente invention concerne de nouvelles poches souples pour le transport de produits fluides bio-pharmaceutiques et son procédé de fabrication.

US-A-5 350 080 décrit des poches utilisables pour des milieux de culture cellulaires ainsi que leur conteneur de transport rigide.

L'industrie bio-pharmaceutique au sens large utilise de plus en plus souvent des poches souples de 20 à 2.000 L et plus, notamment biocompatibles, pour transporter des fluides utilisés dans cette industrie, tels des milieux de cultures, des cultures cellulaires, des solutions tampon, des liquides de nutrition artificielle, des produits sanguins ou dérivés comme le plasma.

Parfois les produits renfermés dans ces poches sont utilisés à des milliers de kilomètres de l'endroit où les poches ont été remplies. Ces produits sont souvent d'une haute valeur financière, voire souvent d'une haute valeur pour la santé des individus puisqu'ils peuvent servir par exemple à la fabrication de médicaments destinés à la santé humaine. Il est donc essentiel que ces poches parviennent sûrement à leur destination, pleines du liquide avec lequel elles ont été remplies à l'origine et non contaminées.

Or ces poches souples sont soumises à de nombreuses contraintes pendant leur transport : accélérations, freinages, ballottements, secousses, vibrations, etc. et donc à de nombreuses forces dont des forces de cisaillement qui ont tendance à altérer le film qui les constitue, notamment aux endroits sensibles comme les pliures. Toutes ces contraintes en conséquence occasionnent fréquemment des affaiblissements, ruptures ou des percements de ces poches.

Il y a lieu de rappeler que ces poches destinées à contenir les milieux et produits liquides précités sont par nature dotées d'un certain nombre d'embouts (ou portes) d'accès permettant par exemple le remplissage, le soutirage, le mélange, etc. de leur contenu, ainsi qu'habituellement un certain nombre de tubulures installées sur tout ou partie de ces portes. Ces tubulures sont elles-mêmes munies souvent d'un ou plusieurs dispositifs en matériau rigide comme des vannes, des filtres ou des clamps qui peuvent contribuer à l'abrasion de la partie supérieure des poches lors de leur transport sur de longues distances. Un percement sur le dessus d'une poche peut être tout aussi sérieux qu'ailleurs par exemple dans le cas du transport d'un contenu stérile.

C'est pourquoi il serait donc souhaitable de disposer d'une poche de transport de liquides bio-pharmaceutiques de volume de 50 litres et plus qui en plus des qualités habituelles de telles poches, c'est-à-dire, biocompatibilité, aptitude à la stérilisation, imperméabilité aux gaz et en particulier à l'oxygène, serait particulièrement résistante au transport sur de longues distances et facile à fabriquer.

On connaît déjà des poches de transport de liquides bio-pharmaceutiques qui sont des poches à soufflet dont les parois, réalisées à partir de trois films séparés, indépendants les uns des autres, sont fabriquées manuellement par sections de soudures.

De telles poches sont par exemple commercialisées par HyClone Laboratories. Elles ont à titre illustratif une contenance de 1 à 1000 litres. Mais lors du transport sur de longues distances, compte tenu des nombreuses contraintes auxquelles sont soumises ces poches, un certain nombre d'entre elles sont perdues à cause de fuites.

US-A-4,968,624 décrit une poche souple de transport de liquide de volume de 20 litres fabriquée à partir d'un film poliol'finique qui définit la chambre intérieure et qui est renforcé au moyen d'un film exterieur laminé.

C'est pourquoi la présente demande a pour objet une poche souple de transport de liquide bio-pharmaceutique de volume de 50 litres et plus, à soufflets, prenant au remplissage une forme sensiblement parallélépipèdique, constituée d'une paroi inférieure, d'une paroi supérieure et de quatre parois latérales caractérisée en ce qu'elle est constituée d'un film unique laminé de 3 couches au moins et formée de préférence à partir de 4 feuilles dudit film unique laminé, la couche interne étant une couche en matière plastique soudable à chaud et biocompatible avec les milieux transportés.

Les liquides bio-pharmaceutiques transportés peuvent être par exemple des milieux de cultures, des cultures cellulaires, des solutions tampon, des liquides de nutrition artificielle, des produits sanguins ou dérivés comme le plasma.

La poche selon l'invention est une poche à soufflets, c'est-à-dire que, placée à plat, deux côtés opposés de la poche sont repliés vers l'intérieur.

Par « film unique laminé » ou « monofilm », l'on entend que la feuille qui constitue les parois de la poche est en apparence un film unique, alors qu'elle a en réalité été réalisée à partir de plusieurs couches de films de nature différente adhérant entre elles.

Selon l'invention, le film unique laminé comprend au moins trois couches et de préférence quatre couches.

La couche interne est une couche en matière plastique soudable à chaud et biocompatible avec les milieux transportés. De telles matières plastiques sont par exemple polyoléfiniques et de préférence le polyéthylène (PE) notamment basse et particulièrement ultra basse densité.

L'épaisseur de cette couche peut aller par exemple de 50 µm à 200 µm et particulièrement de 100 µm à 200 µm.

La couche intermédiaire barrière aux gaz, tels que l'oxygène, le gaz carbonique ou la vapeur d'eau est par exemple réalisée en polyamide (nylon) 6, polyamide 8, polyamide 11, polyamide 12, polyamide 6-6, polyamide 6-10, polyamide -6 ou copolymères polyamide 6 / polyamide 6-6. On peut utiliser aussi des mélanges de résines polymères barrières aux gaz comme un mélange de polyamide ou de polyéthylène et de copolymère éthylène/alcool vinylique (EVOH) en chlorure de polyvinylidène (PVDC). On peut également utiliser une matière plastique ayant subi un traitement de surface à l'oxyde d'aluminum ou à l'oxyde de silicium. Dans des conditions préférentielles de mise en oeuvre, la couche intermédiaire barrière aux gaz est réalisée en copolymère éthylène/alcool vinylique.

L'épaisseur de cette couche peut aller, par exemple, de 6 µm à 20 µm et particulièrement de 10 µm à 20 µm.

La couche externe, de préférence en matière plastique isolante vis à vis d'une soudure thermique, peut être par exemple réalisée en résine polyoléfinique ou polyamide et de préférence en polyester (PET).

L'épaisseur de cette couche peut aller par exemple de 10 µm à 30 µm et particulièrement de 6 µm à 20 µm.

Dans des conditions préférentielles de mise en oeuvre de l'invention, la feuille laminée comprend une quatrième couche, avantageusement placée entre la couche exteme et la couche intermédiaire barrière aux gaz, améliorant la résistance mécanique du film unique laminé.

Cette seconde couche intermédiaire peut être par exemple réalisée en polyoléfine ou en PET et de préférence en polyamide, avantageusement en polyamide 6.

L'adhésif utilisable pour solidariser les différentes couches entre elles est un des adhésifs classiquement utilisables dans le domaine des films laminés constitués de polymères. On utilise de préférence un adhésif époxy, notamment de type polyuréthane-polyester.

Les polymères ci-dessus peuvent être mélangés à des additifs. Par exemple le polyéthylène de la couche interne peut être additionné d'un agent de glissement comme l'érucylamide à la concentration de 600 ppm et/ou un oxyde de silicone à la concentration de 2000 ppm.

Dans des conditions préférentielles de réalisation de l'invention, la poche ci-dessus est réalisée par soudure thermique à plat, à partir de quatre feuilles.

Cette forme parallèlépipèdique peut notamment être obtenue par coupure à 30 à 60° et environ 45° des bords de la feuille par rapport à l'axe vertical de la poche.

Par définition, on appelle "face inférieure" et "face supérieure" de la poche les faces de la poche telles que les soudures se croisent au niveau de ces faces tandis qu'elles sont parallèles entre elles au niveau des faces dites « latérales » de la poche.

Dans d'autres conditions préférentielles de mise en oeuvre de l'invention, la poche ci-dessus comprend à sa face supérieure une collerette de montage de connexions, de préférence allongée, comprenant une ou plusieurs cheminées d'adaptation pour embouts, de préférence identiques, et notamment alignées, comprenant deux lèvres sensiblement cylindriques concentriques.

Ces cheminées d'adaptation peuvent avantageusement coopérer avec des embouts d'accès comprenant une extrémité cylindrique de dimension apte à s'insérer entre les lèvres d'une cheminée. Elles peuvent être au nombre de préférence de 1 à 8 , notamment de 2 à 6, tout particulièrement de 3 à 5.

Dans des conditions préférentielles de réalisation, ces embouts d'accès comprennent en outre une jupe extérieure venant entourer la lèvre extérieure des cheminées d'accès.

Ces embouts d'accès peuvent avoir toutes les configurations classiques telles que notamment des adaptations pour tubulures telles que notamment des adaptations pour tubulure de petit, moyen ou gros diamètre, des bouchons, des connections coudées à angle droit, et ces embouts, notamment ceux pour connexion à angle droit peuvent eux-mêmes comprendre un système à double lèvre analogue à celui des cheminées d'adaptation d'embouts par exemple.

Dans d'autres conditions préférentielles de mise en oeuvre de l'invention, la collerette de montage de connexions est réalisée en résine polyoléfine et de préférence un copolymère éthylène-acétate de vinyle.

Dans encore d'autres conditions préférentielles de mise en oeuvre de l'invention, les embouts sont collés sur les cheminées d'accès à l'aide d'une colle à solvant ou non. Dans toujours d'autres conditions préférentielles, les embouts sont assemblés sur les cheminées d'accès à l'aide d'une colle à solvant ou non ou soudés par un procédé thermique, ou à haute fréquence ou par ultra sons.

La présente invention a encore pour objet une poche ci-dessus munie d'embouts installés sur des cheminées ci-dessus.

Dans d'autres conditions préférentielles de mise en oeuvre de l'invention, tous les matériaux utilisés pour la construction de la poche selon l'invention et de ses accessoires sont capables de supporter une exposition aux radiations ou d'autres techniques connues de stérilisation.

Dans encore d'autres conditions préférentielles de mise en oeuvre de l'invention, la collerette de montage de connexions portant les cheminées d'accès est fixée sur les poches par soudure à chaud.

Dans encore d'autres conditions préférentielles de mise en oeuvre de l'invention, la collerette de montage comprend à sa partie inférieure, opposée aux cheminées d'accès, une ou notamment plusieurs protubérances espacées entre elles pour constituer des canaux, par exemple semi toriques, permettant d'éviter au plateau de se plaquer totalement contre le fond de la poche lors de sa vidange.

Dans encore d'autres conditions préférentielles de mise en oeuvre de l'invention, la collerette de montage comprend des cheminées standardisées, ce qui permet une personnalisation facile des robinets et accessoires de sortie (bouchon, sortie coudée ou non, variété de diamètres, etc.).

Dans encore d'autres conditions préférentielles de mise en oeuvre de l'invention, la face inférieure de la poche est munie d'une bonde par exemple d'évacuation ou de remplissage. Cette bonde d'évacuation ou de remplissage peut être montée sur la poche de la même manière que le plateau ci-dessus. Dans des conditions préférentielles de mise en oeuvre de l'invention, cette bonde comprend une sortie coudée à 90°.

Dans encore d'autres conditions préférentielles de mise en oeuvre de l'invention, la bonde d'évacuation est montée sur une base installée en relief vers l'extérieur du fond de la poche. De préférence, la partie de la bonde venant en relief a une forme polygonale ou une forme quelconque autre que circulaire comme ovale allongé, triangulaire, carrée, hexagonale, etc... Ainsi, cette forme particulière permet un bon centrage de la poche lorsque celle-ci est installée dans un conteneur rigide en vue de son transport, par coopération avec un orifice de forme complémentaire et localisation correspondante prévu au fond du conteneur. Aussi, les coins de la poche peuvent être bien ajustés dans les coins du conteneur, sans risque de torsion de la poche. La bonde peut en même temps constituer un point d'ancrage de la poche au fond du conteneur, ce qui améliore la qualité du transport.

Dans encore d'autres conditions préférentielles de mise en oeuvre, la base proéminente de la bonde est munie d'une gorge périphérique pouvant coopérer avec un clips, de manière à pourvoir être clipsée au moment de son installation dans le conteneur, en vue du remplissage. Ainsi, le placement d'origine de la poche est bien conservé. La présente invention a aussi pour objet un procédé de réalisation d'une poche ci-dessus, caractérisé en ce que l'on amène un film supérieur, un film inférieur, et deux films latéraux repliés sur eux-mêmes, de manière à mettre en contact entre elles et à plat les couches en matière plastique soudables à chaud, en laissant un écart entre les deux films latéraux puis procède à la soudure à chaud des côtés deux par deux, du haut et du fond de la poche et en ce que l'on installe si désiré une collerette (7) de montage de connexions et une bonde (4) d'évacuation.

Dans des conditions préférentielles de mise en oeuvre de l'invention, les soudures du haut et du fond de la poche sont réalisées en K, les branches du K étant inclinées d'environ 45° par rapport à la direction de déplacement des feuilles de monofilm multicouche, par exemple entre 30° et 60° comme on le verra ci-après dans les exemples.

Dans des conditions préférentielles de mise en oeuvre de l'invention, la soudure à chaud est réalisée à l'aide de barres chauffantes. On peut réaliser une seule soudure à un endroit donné. Cependant de préférence on opère une, deux ou notamment trois soudures successives. Dans le cas des soudures en plusieurs étapes, on opère de préférence à des températures différentes.

Dans encore d'autres conditions préférentielles de mise en oeuvre de l'invention, on effectue des soudures d'une largeur comprise entre 5 et 20 mm, de préférence entre 5 et 15 mm et tout particulièrement entre 8 et 15mm.

Les autres opérations, comme la coupure des feuilles au niveau des soudures, sont conventionnelles.

Ainsi, il est possible de procéder en une seule étape à plusieurs opérations de soudure au même niveau, compte tenu de la présence de la couche externe formant isolation vis-à-vis d'une soudure thermique. Ainsi, seules les couches internes font l'objet d'une soudure entre elles.

L'invention sera mieux décrite si l'on se réfère aux dessins annexés sur lesquels :

La figure 1 est une vue en perspective d'une poche selon l'invention dans la configuration qu'elle prend lorsqu'elle est remplie, avant montage d'une bonde et d'une collerette de montage d'embouts.

La figure 2 représente une vue de dessus d'une poche à plat avant montage d'une bonde et d'une collerette de montage d'embouts.

La figure 3 représente une vue en perspective du placement des 4 films et de deux barres chauffantes longitudinales (pour soudure d'un côté d'une poche), lors de la fabrication d'une poche.

La figure 4 représente une vue de face des films et de deux barres chauffantes transversales (pour soudure du fond ou du haut d'une poche) lors de la fabrication d'une poche.

La figure 5 représente une vue de dessus de l'emplacement des soudures côté haut ou fond d'une poche.

### EXEMPLE 1 : Fabrication d'un film quadricouches

On a réalisé par collage un film laminé de la structure suivante :

| Nature de la résine | Epaisseur |
|---|---|
| PET | 12 µm |
| Adhésif époxy polyuréthane-polyester | 2,5 µm |
| PA 6 | 15 µm |
| Adhésif époxy polyuréthane-polyester | 2,5 µm |
| EVOH | 12 µm |
| Adhésif époxy polyuréthane-polyester | 2,5 µm |
| PE ultra basse densité | 150 µm |

### EXEMPLE 2 : Fabrication d'une poche

Sur une machine automatique conçue à cet effet, 4 bobines de film plastique de l'exemple 1 sont déroulées pour constituer les 4 parois d'une poche cubique ou parallélépipèdique : deux films sont tendus à plat (au-dessus et en dessous) et deux films sont conformés en soufflets à plat et insérés entre les deux premiers.

Les quatre soudures longitudinales sont opérées par succession de trois soudures de type thermique sur la même section de 30 à 90 cm en fonction d'une avance prédéterminée du film. Chacune de ces trois soudures est réalisée à une température différente.

Ce procédé garantit
- la solidité des soudures, et
- un recouvrement minimum des sections de soudures des films par les barres de soudure permettant de varier ainsi la hauteur de la poche.

Les soudures des bas et haut de la poche sont réalisées l'une après l'autre suivant le même procédé au minimum d'une soudure et préférentiellement de trois soudures successives à températures différentes.

Ces soudures des bas et haut de la poche sont réalisées par un ensemble de barres de soudures situées :
- d'une part, à 90° par rapport à l'avancement du film, ceci permet de souder une section comprenant 4 + 2 + 4 épaisseurs de film (soudure dite "transversale"),
- d'autre part, selon un angle compris entre 30° et 60° par rapport à l'avancement du film afin de conformer exactement la poche déployée en trois dimensions à la géométrie du fond et des côtés d'un conteneur rigide dans laquelle elle sera placée (soudure dite "en K").

Les soudures transversales et en K ci-dessus sont réalisées à l'exacte intersection des deux films des soufflets avec les deux films plats. On assure ainsi l'étanchéité et la résistance de la poche.

Entre les opérations de soudure du haut et du bas de la poche (transversale et en K) on réalise les trois opérations complémentaires suivantes destinées à donner à la poche des fonctionnalités souhaitées de remplissage et de vidange par le haut et par le bas :
1) poinçonnage du film plat supérieur et/ou inférieur,
2) Installation d'une collerette pour embout et/ou pour bonde,
3) soudure thermique du/des film(s) plat(s) sur cet embout et/ou bonde.

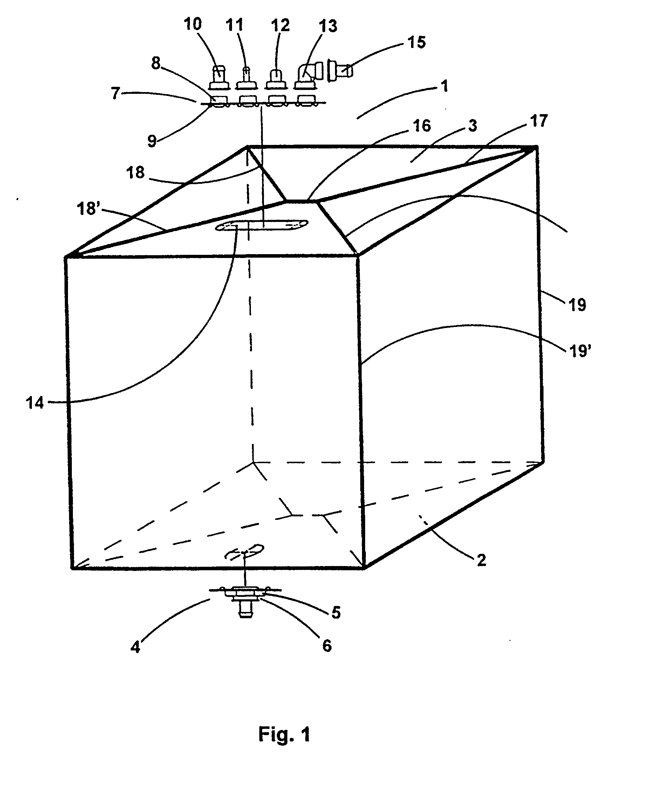

Sur la figure 1, on distingue une poche 1 souple selon l'invention comprenant une paroi inférieure 2, une paroi supérieure 3, et quatre parois latérales. La paroi inférieure 2 est munie d'une bonde 4 permettant la vidange du liquide bio-pharmaceutique qu'elle contient. Cette bonde comprend une base en relief 5 ici de forme hexagonale, pouvant coopérer avec un orifice de forme complémentaire prévu au fond d'un conteneur rigide de transport pour poches. Cette base est munie d'une gorge 6 permettant l'installation d'un clips pour bloquer la bonde vis-à-vis du conteneur, ce dernier étant pris en sandwich entre la collerette de montage de la base de la bonde et le clips.

Sur la face supérieure 3 du conteneur 1, on a installé une collerette 7 de montage de connexions ici représentée en position non montée sur la poche. Cette collerette comprend 4 cheminées 8 identiques formées de 2 lèvres concentriques. Chacune des cheminées comprend, du côté intérieur de la poche, quatre protubérances semi-toriques 9 espacées les unes des autres pour ménager un passage entre deux protubérances 9 successives. On peut installer sur ces cheminées un certain nombre d'embouts 10, 11, 12, 13 qui sont ici respectivement des embouts pour tubulure de gros diamètre, pour tubulure de petit diamètre, un bouchon, un coude à 90° comprenant lui-même une cheminée de structure analogue à celle des précédentes sur laquelle on peut installer un autre embout 15, ici de structure analogue à celle de l'embout pour tubulure de gros diamètre 10.

Sur cette figure, on peut également observer en particulier sur la face supérieure et sur la face inférieure la structure des soudures, tout d'abord une soudure 16 transversale à la direction d'avancement des films lors de la fabrication de la poche, ainsi que des soudures inclinées par rapport à cet axe, soudures 17 et 17' d'une part (effectuées ensemble), et soudures 18 et 18' (effectuées elles aussi ensemble). Les soudures latérales 19 et 19' sont elles aussi réalisées ensemble, et il en est de même des soudures qui leur sont parallèles sur la face opposée.

On remarque enfin un orifice 14 ménagé sur la paroi 3 supérieure de la poche pour le montage de la collerette 7. La paroi 2 inférieure de la poche est également munie d'un orifice pour le montage de la bonde 4.

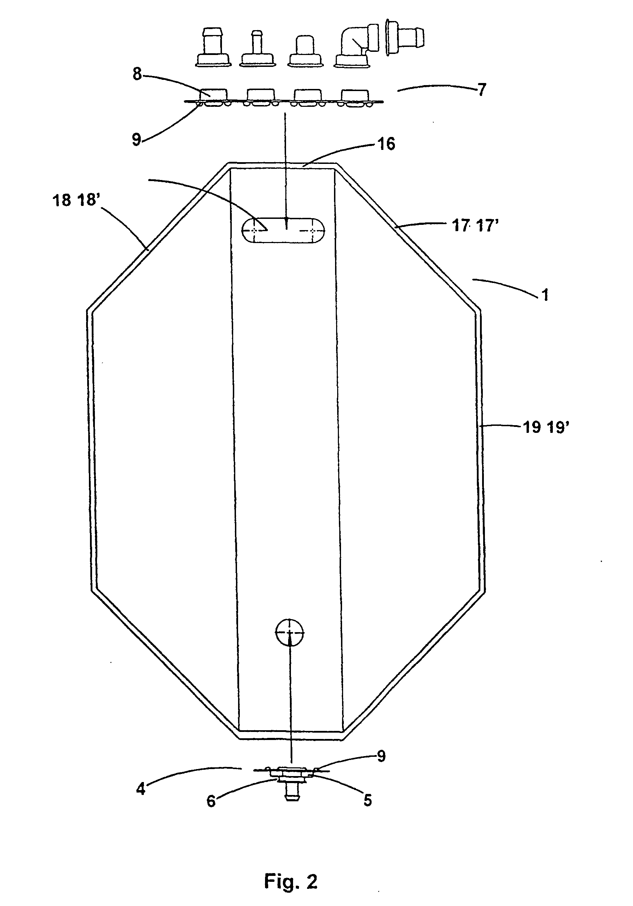

Sur la figure 2, on distingue les mêmes éléments que sur la figure 1, à ceci près que la poche est dans ce cas installée à plat, telle qu'on l'obtient juste après sa fabrication. Ainsi, on observe que les soudures 17 et 17' sont superposées, et qu'il en est de même pour les soudures 18, 18' et 19, 19'.

Sur cette figure, la collerette 7 et la bonde 4 ont été représentées agrandies par rapport à la poche 1. On peut également faire observer que dans la partie centrale de la poche, où l'on peut observer les orifices prévus pour la collerette et l'embout, on a seulement deux épaisseurs de films laminés, tandis que sur les côtés, l'on a quatre épaisseurs de ce film.

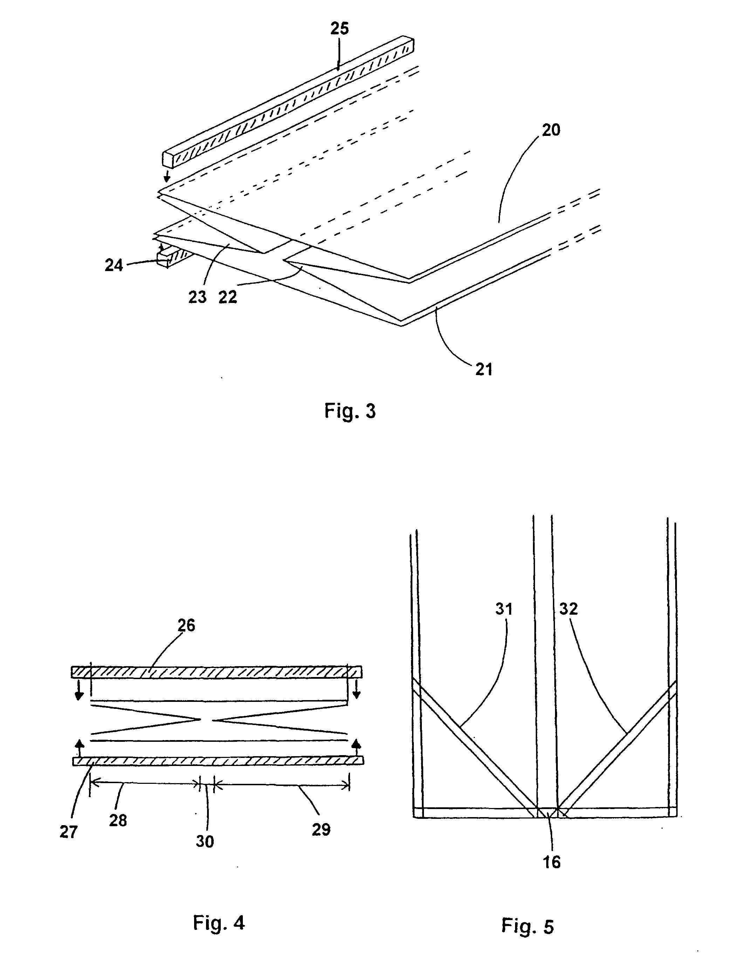

Sur la figure 3, qui illustre la fabrication d'une poche selon l'invention, on observe la manière dont les films sont alimentés. Deux films 20 et 21 sont tendus à plat, l'un supérieur et l'un inférieur, et deux films 22 et 23 sont conformés en soufflets à plat et insérés pliés entre les deux premiers. Deux barres chauffantes 24 et 25 prennent en sandwich les quatre épaisseurs de films provenant de trois films 20, 21, 23 différents pour produire simultanément deux soudures. Il en est de même pour le côté opposé (on n'a pas représenté les barres chauffantes du côté opposé).

Sur la figure 4, on peut observer des barres 26 et 27 qui procureront les soudures transversales du haut et du bas de la poche référencées en 16 sur les figures 1 et 2.

On peut observer, comme évoqué ci-dessus, que dans cette technique de fabrication, on peut considérer deux zones 28 et 29 dans lesquelles on trouve quatre épaisseurs du film constituant la poche, tandis que dans une zone intermédiaire 30 on n'a que deux épaisseurs. Malgré cela, compte tenu de la structure particulière des films selon l'invention, on obtient par fabrication à plat des soudures et des poches d'une grande solidité, même au transport sur de longues distances.

Sur la figure 5, on observe le principe de la soudure dite en K comprenant, d'une part, une soudure perpendiculaire à l'avancement des films comme représenté sur la figure 4 et conduisant à la soudure 16 ainsi que deux soudures inclinées 31 et 32 correspondant à celles illustrées en 17,17' et 18, 18' sur les figures 1 et 2.

Une fois que les opérations de soudure sont réalisées, on peut bien évidemment couper les parties externes des films au delà des zones de soudure.

## Revendications

1. Une poche souple (1) de transport de liquide bio-pharmaceutique de volume de 50 litres et plus, à soufflets, prenant au remplissage une forme sensiblement parallélépipèdique, constituée d'une paroi inférieure (2), d'une paroi supérieure (3) et de quatre parois latérales **caractérisée en ce qu'**elle est fabriquée à partir de 4 feuilles d'un film unique laminé de 3 couches au moins, la couche interne étant une couche en matière plastique soudable à chaud et biocompatible avec les milieux transportés.

2. Une poche souple selon la revendication 1, **caractérisée en ce que** le film unique laminé comprend quatre couches.

3. Une poche souple Une poche souple selon l'une des revendications 1 et 2, **caractérisée en ce que** la couche interne est en polyéthylène (PE) dont l'épaisseur est de 50 µm à 200 µm.

4. Une poche souple selon l'une des revendications 1 à 3, **caractérisée en ce que** la couche intermédiaire barrière aux gaz, tels que l'oxygène, le gaz carbonique ou la vapeur d'eau est en copolymère éthylène/alcool vinylique dont l'épaisseur est de 6 µm à 20 µm.

5. Une poche souple selon l'une des revendications 1 à 4 **caractérisée en ce que** la couche externe est en polyester (PET) dont l'épaisseur est de 6 µm à 20 µm.

6. Une poche souple selon l'une des revendications 1 à 5 **caractérisée en ce qu'**elle comprend une quatrième couche, améliorant la résistance mécanique du film unique laminé.

7. Une poche souple selon la revendication 6 **caractérisée en ce que** la quatrième couche est une seconde couche intermédiaire réalisée en polyamide.

8. Une poche souple selon l'une des revendications 1 à 7 **caractérisée en ce qu'**elle est réalisée par soudure thermique à plat, à partir de quatre feuilles.

9. Une poche souple selon l'une des revendications 1 à 8 **caractérisée en ce qu'**elle comprend à sa face supérieure une collerette (7) de montage de connexions, comprenant une ou plusieurs cheminées (8) d'adaptation pour embouts (10,11,12,13), lesdites cheminées (8) comprenant deux lèvres sensiblement cylindriques concentriques.

10. Une poche souple selon la revendication 9 **caractérisée en ce qu'**elle est munie d'embouts (10,11,12,13) installés sur les cheminées (8).

11. Une poche souple selon l'une des revendications 1 à 10 **caractérisée en ce que** la face inférieure de la poche (2) est munie d'une bonde (4) d'évacuation.

12. Une poche souple selon la revendication 11 **caractérisée en ce que** la bonde (4) d'évacuation est montée sur une base (5) installée en relief vers l'extérieur du fond de la poche, la partie de la bonde venant en relief (5) ayant une forme polygonale ou une forme quelconque autre que circulaire.

13. Un procédé de réalisation d'une poche telle que définie à l'une des revendications 1 à 12, **caractérisé en ce que** l'on amène un film supérieur (20), un film inférieur(21), et deux films latéraux (22,23), repliés sur eux-mêmes, de manière à mettre en contact entre elles et à plat les couches en matière plastique soudables à chaud, en laissant un écart entre les deux films latéraux (22,23), puis procède à la soudure à chaud
- des côtés deux par deux,
- du haut et du fond de la poche,
et **en ce que** les soudures du haut (16), et du fond de la poche sont réalisées en K, les branches du K (31,32), étant inclinées entre 30° et 60° par rapport à la direction de déplacement des feuilles de monofilm multicouche.

14. Un procédé selon la revendication 13, **caractérisé en ce que** l'on opère une, deux ou trois soudures successives à l'emplacement d'une soudure donnée.

## Patentansprüche

1. Elastischer Beutel (1) für den. Transport einer biopharmazeutischen Flüssigkeit mit einem Fassungsvermögen von mindestens 50 Litern und weiters mit Faltenbälgen, welcher beim Füllen im Wesentlichen die Form eines Parallelepipeds annimmt und durch eine untere Wand (2), eine obere Wand (3) und vier Seitenwände gebildet ist, **dadurch gekennzeichnet, dass** er aus vier Blättern einer aus mindestens drei Schichten laminierten Kombifolie hergestellt ist, wobei die Innenschicht eine Schicht aus warmschweißbarem Kunststoff ist, die mit den transportierten Medien bioverträglich ist.

2. Elastischer Beutel nach Anspruch 1, **dadurch gekennzeichnet, dass** die laminierte Kombifolie vier Schichten aufweist.

3. Elastischer Beutel nach einem der Ansprüche 1 und 2, **dadurch gekennzeichnet, dass** die Innenschicht aus Polyethylen (PE) mit einer Dicke von 50 µm bis 200 µm besteht.

4. Elastischer Beutel nach einem der Ansprüche 1 bis 3, **dadurch gekennzeichnet, dass** die Zwischenschicht, die eine Isolierschicht gegen Gase wie Sauerstoff, Kohlendioxid oder Wasserdampf darstellt, aus einem Ethylen/Vinylalkohol-Copolymer mit einer Dicke von 6 µm bis 20 µm besteht.

5. Elastischer Beutel nach einem der Ansprüche 1 bis 4, **dadurch gekennzeichnet, dass** die Außenschicht aus Polyester (PET) mit einer Dicke von 6 µm bis 20 µm besteht.

6. Elastischer Beutel nach einem der Ansprüche 1 bis 5, **dadurch gekennzeichnet, dass** er eine vierte Schicht zur Verbesserung der mechanischen Festigkeit der laminierten Kombifolie aufweist.

7. Elastischer Beutel nach Anspruch 6, **dadurch gekennzeichnet, dass** die vierte Schicht eine aus Polyamid gefertigte zweite Zwischenschicht ist.

8. Elastischer Beutel nach einem der Ansprüche 1 bis 7, **dadurch gekennzeichnet, dass** er aus vier Blättern durch Flachwarmschweißung hergestellt ist.

9. Elastischer Beutel nach einem der Ansprüche 1 bis 8, **dadurch gekennzeichnet, dass** er an seiner Oberseite einen Kragen (7) zur Befestigung von Anschlüssen aufweist, der einen oder mehrere Stutzen (8) zur Anbringung von Ansatzstücken (10, 11, 12, 13) enthält, wobei die Stutzen (8) zwei konzentrische, im Wesentlichen zylindrische Lippen aufweisen.

10. Elastischer Beutel nach Anspruch 9, **dadurch gekennzeichnet, dass** er mit Ansatzstücken (10, 11, 12, 13) versehen ist, die an den Stutzen (8) angeordnet sind.

11. Elastischer Beutel nach einem der Ansprüche 1 bis 10, **dadurch gekennzeichnet, dass** die Unterseite des Beutels (2) mit einer Ausflussöffnung (4) versehen ist.

12. Elastischer Beutel nach Anspruch 11, **dadurch gekennzeichnet, dass** die Ausflussöffnung (4) an einer Basis (5) vorgesehen ist, die vom Boden des Beutels nach außen vorstehend angebracht ist, wobei der vorstehende Teil (5) der Öffnung polygonale Form oder irgendeine andere Form als die eines Kreises hat.

13. Verfahren zur Herstellung eines Beutels nach einem der Ansprüche 1 bis 12, **dadurch gekennzeichnet, dass** eine obere Folie (20), eine untere Folie (21) und zwei seitliche Folien (22, 23), die übereinander gestülpt sind, derart zugeführt werden, dass die Schichten aus warmschweißbarem Kunststoff flach miteinander in Kontakt gebracht werden, wobei zwischen den beiden seitlichen Folien (22, 23) ein Abstand gelassen wird, und dann die Warmschweißung
- der Seiten paarweise,
- des Oberteils und des Bodens des Beutels
vorgenommen wird, und dass die Schweißung des Oberteils (16) und des Bodens des Beutels K-förmig erfolgt, wobei die Schenkel des K (31, 32) zwischen 30° und 60° zur Verschieberichtung der Lagen der mehrschichtigen Monofolie geneigt sind.

14. Verfahren nach Anspruch 13, **dadurch gekennzeichnet, dass** eine, zwei oder drei aufeinander folgende Schweißungen anstelle einer gegebenen Schweißung ausgeführt werden.

## Claims

1. A flexible sachet (1) for transporting bio-pharmaceutical liquids with a volume of 50 litres and more, of the bellows type, assuming when filled a substantially parallelepiped shape, comprised of a bottom wall (2), a top wall (3) and four lateral walls, **characterised in that** it is made from 4 sheets of a single laminated film with at least 3 layers, the internal layer being a layer of plastic material that can be heat welded and is biocompatible with the media transported.

2. A flexible sachet according to claim 1, **characterised in that** the single laminated film comprises 4 layers.

3. A flexible sachet according to one of claims 1 and 2, **characterised in that** the internal layer is of polyethylene (PE) and is 50 µm to 200 µm thick.

4. A flexible sachet according to one of claims 1 to 3, **characterised in that** the intermediate layer which is a barrier to gases, such as oxygen, carbon dioxide or water vapour is made of ethylene/vinyl alcohol copolymer and is 6 µm to 20 µm thick.

5. A flexible sachet according to one of claims 1 to 4, **characterised in that** the external layer is made of polyester (PET) and is 6µm to 20µm thick.

6. A flexible sachet according to one of claims 1 to 5, **characterised in that**.it comprises a fourth layer, increasing the mechanical strength of the laminated single layer.

7. A flexible sachet according to claim 6 **characterised in that** the fourth layer is a second intermediate layer made of polyamide.

8. A flexible sachet according to one of claims 1 to 7, **characterised in that** it is made by heat welding flat, from four sheets.

9. A flexible sachet according to one of claims 1 to 8, **characterised in that** it comprises on its top face a flange (7) for mounting connectors, comprising one or more adapting chimneys (8) for connectors (10,11,12,13), said chimneys (8) comprising two substantially cylindrical concentric lips.

10. A flexible sachet according to claim 9 **characterised in that** it is provided with connectors (10,11,12,13) installed on the chimneys (8).

11. A flexible sachet according to one of claims 1 to 10, **characterised in that** the bottom face of the sachet (2) is provided with a drain bung (4).

12. A flexible sachet according to claim 11 **characterised in that** the drain bung (4) is mounted on an outwardly upstanding base (5) on the bottom of the sachet, the upstanding part of the bung (5) having a polygonal form or non-circular form.

13. A process for manufacturing a sachet as defined in one of claims 1 to 12, **characterised in that** a top film (20), a bottom film (21), and two lateral films (22, 23), folded over onto themselves, in such a way as to place the layers of plastic heat weldable material flat and in contact with each other (22,23) then heat welding is carried out
- of the sides two by two,
- of the top and bottom of the sachet,
and **in that** the welding of the top (16) and the bottom of the sachet is carried out in a K shape, the arms of the K (31,32), at an angle of 30° and 60° in relation to the direction of displacement of the sheets of multilayer monofilm.

14. A process according to claim 13, **characterised in that** welding is carried out two or three times successively at a given point of welding.
